(19) Europäisches Patentamt
European Patent Office
Office européen des brevets

(11) EP 1 477 153 A1

(12) **DEMANDE DE BREVET EUROPEEN**

(43) Date de publication:
**17.11.2004 Bulletin 2004/47**

(51) Int Cl.[7]: **A61K 7/02**, A61K 7/48, A61K 7/025

(21) Numéro de dépôt: **04290462.3**

(22) Date de dépôt: **20.02.2004**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **25.02.2003 FR 0350035**

(71) Demandeur: **L'OREAL**
**75008 Paris (FR)**

(72) Inventeurs:
- **Blin, Xavier**
  **75015 Paris (FR)**
- **Lebre, Caroline**
  **94320 Thiais (FR)**
- **Lion, Bertrand**
  **95270 Luzarches (FR)**

(74) Mandataire: **Allab, Myriam**
**L'OREAL - D.I.P.I.**
**25-29 Quai Aulagnier**
**92600 Asnières (FR)**

(54) **Composition cosmétique comprenant une dispersion de particules de polymères et un composé plastifiant le polymère**

(57) La présente invention se rapporte à une composition, notamment cosmétique pour le soin ou le maquillage de la peau et/ou des phanères et/ou des lèvres, comprenant un milieu physiologiquement acceptable contenant a) au moins une phase grasse liquide, b) des particules de polymère dispersées dans ladite phase grasse, et c) au moins un composé choisi parmi les esters des acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère.

La présente invention se rapporte également à une composition, notamment cosmétique pour le soin ou le maquillage de la peau et/ou des phanères et/ou des lèvres, comprenant un milieu physiologiquement acceptable contenant a) au moins une phase grasse liquide, b) des particules de polymère dispersées dans ladite phase grasse, et c) au moins un composé ayant un paramètre de solubilité $\delta_h$ compris entre 5,5 et 11 $(J/cm^3)^{1/2}$, ledit composé étant présent en une quantité suffisante pour plastifier ledit polymère.

Cette composition permet d'obtenir sur la peau, les phanères ou les lèvres un film qui ne transfère pas, de bonne tenue et qui se dépose facilement.

L'invention se rapporte également à un procédé de soin ou de maquillage de la peau, des phanères et des lèvres utilisant une telle composition.

EP 1 477 153 A1

## Description

La présente invention a trait à une composition destinée en particulier au domaine cosmétique contenant des particules de polymère dispersées dans une phase grasse liquide, et un composé qui plastifie ledit polymère.

Plus précisément, l'invention se rapporte à une composition de soin ou de maquillage des matières kératiniques, comme la peau aussi bien du visage que du corps humain, y compris du cuir chevelu, les phanères comme les cils, les sourcils, les ongles et les cheveux mais aussi des lèvres, les paupières inférieures ou supérieures des êtres humains.

La composition selon l'invention permet d'obtenir un maquillage de bonne tenue, qui ne transfère pas et/ou facile à appliquer.

On entend par compositions "non transfert" ou "sans transfert", des compositions qui forment, sur un support, un dépôt qui ne se dépose pas, au moins en partie, sur les objets (verre, vêtements, cigarette) avec lesquels elles sont mises en contact.

On entend par maquillage de bonne tenue, un maquillage dont la couleur et/ou la brillance persiste au cours de la journée, et qui résiste aux épreuves, en particulier au repas dans le cas d'un rouge à lèvres.

La composition est facile à appliquer, en ce sens qu'elle est glissante à l'application en se délitant facilement, et/ou que la masse de son dépôt en un passage est suffisante pour obtenir un maquillage satisfaisant.

Selon un de ses aspects, la présente invention se rapporte également à un produit cosmétique comprenant au moins deux compositions qui sont appliquées successivement, notamment sur la peau, sur les lèvres ou sur les phanères, la première composition contenant i) une phase grasse liquide, ii) des particules de polymère dispersées dans ladite phase grasse liquide et iii) un composé présent en une quantité suffisante pour plastifier ledit polymère, et la deuxième composition comprenant un milieu physiologiquement acceptable.

Le demandeur a envisagé la fabrication de compositions de maquillage contenant des particules de polymère dispersées et stabilisées en surface par un stabilisant dans une phase grasse liquide, telles que décrites dans le document EP 1 249 223 (L'OREAL). Ce document suggère associer un plastifiant au polymère, lorsque le polymère présente une température de transition vitreuse trop élevée pour l'application souhaitée. Les agents plastifiants proposés sont ceux cités dans le document FR-A-2782 917.

Or, le demandeur a constaté qu'en choisissant un plastifiant particulier dont les paramètres de solubilité ont été sélectionnés, on obtient des compositions contenant des particules de polymère dispersées dans une phase grasse liquide dont la tenue et/ou le non-transfert et/ou la faculté de dépôt sont améliorés.

En particulier, le produit de l'invention permet l'obtention de dépôts continus non collants, de bonne couvrance, ne migrant pas et ne desséchant pas la peau ou les lèvres sur lesquelles. il est appliqué, aussi bien lors de l'application qu'au cours du temps. Il présente, en outre de bonnes propriétés d'application, puisque le dépôt sèche rapidement et que la couleur se dépose correctement en un seul passage. Le produit permet d'obtenir un maquillage naturel aux contours nets qui laisse une sensation de lèvres nues, contrairement aux compositions de l'art antérieur pour lesquelles la consommatrice perçoit une rétractation des lèvres lors du dépôt, un marquage inesthétique du microrelief des lèvres et une sensation de dessèchement.

Le demandeur a de plus constaté que le produit selon l'invention présente des qualités d'étalement et d'adhésion sur la peau, les lèvres, les cils ou les muqueuses, particulièrement intéressantes, ainsi qu'un toucher onctueux et agréable.

Les propriétés de tenue, de non transfert, et de non migration en font un produit particulièrement approprié pour réaliser des produits de maquillage de la peau, en particulier des lèvres tels que rouges à lèvres, brillants à lèvres ou des yeux tels que mascara, eye-liners, fards à paupières, ou de la peau tels que des fonds de teint.

L'invention s'applique non seulement aux produits de maquillage des lèvres mais aussi aux produits de soin et/ou de traitement des lèvres comme les baumes, de la peau, y compris du cuir chevelu, comme les crèmes de soin journalier et de protection solaire de la peau du visage, aux produits de maquillage de la peau, aussi bien du visage que du corps humain, comme les fonds de teints notamment coulés en stick ou en coupelle, les produits anti-cerne et les produits de tatouage éphémère, aux produits d'hygiène corporelle comme les déodorants notamment en stick, et aux produits de maquillage des yeux comme les eye-liners en particulier sous forme de crayon et les mascaras notamment sous forme de pain.

### Définitions

Par "phase grasse liquide", au sens de la demande, on entend tout milieux non aqueux liquide à température ambiante (25°C) et pression atmosphérique (760 mm de Hg), composé d'un ou plusieurs corps gras liquides à température ambiante, appelés aussi huiles, à l'exception de l'ester d'acide et de polyol, ainsi que des gélifiants et des stabilisants des pigments éventuellement présents dans la composition, lorsqu'ils sont liquides à température ambiante et pression atmosphérique. Cette phase grasse peut contenir une phase grasse liquide volatile et/ou une phase grasse

non volatile.

Par "phase grasse non volatile", on entend tout milieu susceptible de rester sur la peau ou les lèvres sans s'évaporer. Une phase grasse non volatile a en particulier une pression de vapeur à température ambiante et pression atmosphérique, non nulle inférieure à 0,02 mm de Hg et mieux inférieur à $10^{-3}$ mm de Hg.

Par "phase grasse volatile", on entend tout milieu non aqueux susceptible de s'évaporer de la peau ou des lèvres, à température ambiante et pression atmosphérique. Cette phase volatile comporte notamment des huiles ayant une pression de vapeur, à température ambiante (25°C) et pression atmosphérique (760mm Hg) allant de $10^{-3}$ à 300 mm de Hg (0,13 Pa à 40 000 Pa), en particulier allant de 0,02 à 300 mm de Hg (2,66 Pa à 40 000 Pa).

Par "huile hydrocarbonée", on entend des huiles contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements éther, ester ou acide carboxylique.

Par "substitué" on entend comprenant en outre au moins un substituant choisi parmi un atome d'oxygène, d'azote ou d' halogène, un groupement hydroxyle, éther, oxyalkylène, polyoxyalkylène, carboxyle, amine ou amide.

Par "saccharide" on entend un mono ou un polysaccharide.

Les "groupes polaires" sont bien connus de l'homme du métier ; il peut s'agir par exemple des groupes polaires ioniques ou non ioniques choisis parmi -COOH ; -OH ; oxyde d'éthylène; oxyde de propylène; $-PO_4$ ; -NHR ; $-NR_1R_2$ avec $R_1$, $R_2$ formant éventuellement un cycle et représentant un radical alkyle ou alkoxy linéaire ou ramifié en $C_1$ à $C_{20}$.

Par "agent gélifiant", on entend un composé qui augmente la viscosité du milieu dans lequel il est incorporé, ou qui rigidifie ledit milieu. L'agent gélifiant selon l'invention n'inclut pas les cires.

Une cire, au sens de la présente invention, est un composé gras lipophile, solide à température ambiante (25°C), à changement d'état solide/liquide réversible, ayant une température de fusion supérieure à 40°C pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. C'est cette recristallisation dans le mélange qui est responsable de la diminution de la brillance dudit mélange.

Par "composé qui plastifie le polymère", on entend un composé organique présent dans une quantité suffisante pour abaisser la température de transition vitreuse du polymère selon l'invention d'au moins 2°C, d'au moins 3°C ou d'au moins 4 °C, de préférence d'au moins 5°C.

La température de transition vitreuse du polymère selon l'invention est mesurée à l'aide d'un calorimètre à balayage différentiel, par exemple le calorimètre vendu sous la dénomination MDSC 2920 par la société TA Inst., par la technique dite de l'analyse calorimétrique à compensation de puissance ("Differential Scanning-Calorimetry" ou DSC en anglais), dans laquelle on applique par exemple une montée en température de 2°C par minute.

Pour évaluer l'abaissement de la température de transition vitreuse de la température de transition vitreuse provoqué par le composé, on commence par mesurer la température de transition vitreuse du polymère pur par la méthode ci-dessus, puis on prépare un mélange du polymère et du composé et on effectue la mesure par la même méthode dans les mêmes conditions.

L'abaissement de la température de transition vitreuse est fonction de la quantité de composé plastifiant dans la composition. Cette quantité doit être telle qu'elle soit suffisante pour provoquer une diminution, un abaissement effectif de la température de transition vitreuse du polymère. Cette diminution, cet abaissement est d'au moins 2°C, de préférence d'au moins 4°C, de préférence encore d'au moins 5°C.

**Résumé de l'invention**

Un premier objet de l'invention est une composition cosmétique destinée à être appliquée sur les matières kératiniques, comprenant a) au moins une phase grasse liquide, b) des particules d'au moins un polymère dispersées dans ladite phase grasse liquide, et c) au moins un composé ayant un paramètre de solubilité $\delta_h$ compris entre 5,5 et 11 $(J/cm^3)^{1/2}$, ledit composé étant présent en une quantité suffisante pour plastifier ledit polymère.

Un autre objet de l'invention est une composition cosmétique destinée à être appliquée sur les matières kératiniques, comprenant a) au moins une phase grasse liquide, b) des particules d'au moins un polymère dispersées dans ladite phase grasse liquide, et c) au moins un composé choisi parmi les esters des acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère.

Un autre objet de l'invention est l'utilisation - dans une composition destinée à être appliquée sur la peau, les lèvres et les phanères cosmétiques ou hygiéniques ou pour la fabrication d'une composition dermatologique ou pharmaceutique à application sur la peau, les lèvres et les phanères, ladite composition contenant des particules d'au moins un polymère dispersées dans une phase grasse liquide - d'au moins un composé choisi parmi les esters des

acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère, pour limiter le transfert de la composition et/ou augmenter sa tenue dans le temps et/ou améliorer son dépôt.

L'invention a encore pour objet un procédé cosmétique de soin ou de maquillage des matières kératiniques et notamment des lèvres, des phanères ou de la peau, consistant à appliquer sur les lèvres, les phanères ou la peau une composition cosmétique telle que définie précédemment.

L'invention a encore pour objet un procédé pour limiter le transfert d'une composition de maquillage ou de soin de la peau ou des lèvres et/ou augmenter sa tenue dans le temps et/ou faciliter son dépôt, ladite composition contenant une phase grasse liquide et des particules de polymère dispersées dans la phase grasse, ledit procédé consistant à introduire dans la phase grasse au moins un composé choisi parmi les esters des acides polycarboxyliques aliphatiques ou aromatiques et des alcools aliphatiques ou aromatiques comprenant de 1 à 10 atomes de carbone, ledit composé étant en quantité suffisante pour plastifier le polymère.

L'invention a encore pour objet un procédé de fabrication d'une composition cosmétique, consistant à introduire dans un milieu physiologiquement acceptable liquide a) une dispersion de particules de polymère dans une phase grasse liquide, et b) au moins un composé choisi parmi les esters des acides polycarboxyliques aliphatiques ou aromatiques et des alcools aliphatiques ou aromatiques comprenant de 1 à 10 atomes de carbone, ledit composé étant en quantité suffisante pour plastifier le polymère.

**Polymère en dispersion**

Selon l'invention le polymère est un solide insoluble dans la phase organique liquide de la première composition même à sa température de ramollissement, à l'inverse d'une cire même d'origine polymérique qui est elle soluble dans la phase organique liquide (ou phase grasse) à sa température de fusion. Il permet, en outre, la formation d'un dépôt notamment filmogène continu et homogène et/ou est caractérisé par l'enchevêtrement des chaînes polymériques. Avec une cire même obtenue par polymérisation on obtient, après fusion dans la phase organique liquide, une recristallisation. Cette recristallisation est en particulier responsable de la perte de la brillance de la composition.

Pour avoir des propriétés de sans transfert optimales, on choisit la quantité de polymère en fonction de la quantité de matières colorantes et/ou d'actifs et/ou d'huiles, contenue dans la première composition. En pratique, la quantité de polymère peut être supérieure à 5 % en poids (en matière active), par rapport au poids total de la composition.

Un avantage de l'utilisation d'une dispersion de particules de polymère dans une composition de l'invention est que ces particules restent à l'état de particules élémentaires, sans former d'agglomérats, dans la phase grasse. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion de polymère est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau, les lèvres ou les phanères.

La première composition du produit selon l'invention comprend donc avantageusement au moins une dispersion stable de particules de polymère généralement sphériques d'un ou plusieurs polymères, dans une phase organique liquide physiologiquement acceptable. Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500nm. Il est toutefois possible d'obtenir des tailles de particules de polymère allant jusqu'à 1 µm.

De préférence, les particules de polymères en dispersion sont insolubles dans les alcools hydrosolubles tels que, par exemple, l'éthanol.

Les polymères en dispersion utilisables dans la première composition de l'invention ont de préférence un poids moléculaire de l'ordre de 2000 à 10 000 000 g/mol.

Il est possible d'utiliser des polymères filmifiables, de préférence ayant une Tg basse, inférieure ou égale à la température de la peau et notamment inférieure ou égale à 40°C.

De préférence le polymère utilisé est filmifiable, c'est-à-dire apte à former seul ou en association avec un agent plastifiant, un film isolable. Il est toutefois possible d'utiliser un polymère non filmifiable.

Par « polymère non filmifiable », on entend un polymère non capable de former, seul, un film isolable. Ce polymère permet, en association avec un composé non volatil du type huile, de former un dépôt continu et homogène sur la peau et/ou les lèvres.

Parmi les polymères filmifiables, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Parmi les polymères non filmifiables, on peut citer des homopolymères ou copolymères radicalaires, vinyliques ou acryliques, éventuellement réticulés, ayant de préférence une Tg supérieure à 40°C et notamment allant de 45° à 150°C, utilisés seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phthalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés. comme les polyuréthanes ou acryliques siliconés, polymères fluorés et leurs mélanges.

Le ou les polymères en dispersion dans la phase liquide organique peuvent représenter en matière sèche de 5 à 40% du poids de la composition, de préférence de 5 à 35 % et mieux de 8 à 30 %.

Selon un mode de mise en oeuvre, les particules de polymère en dispersion sont stabilisées en surface par un stabilisant solide à température ambiante. Dans ce cas, la quantité en matière sèche de la dispersion représente

la quantité totale de polymère + stabilisant, sachant que la quantité de polymère ne peut être inférieure à 5 %.

On choisit de préférence d'utiliser une dispersion de polymère filmifiable, les particules étant dispersées dans une huile volatile.

**Composé plastifiant le polymère**

Les polymères en dispersion utilisables dans la première composition de l'invention ont de préférence une température de transition vitreuse (Tg) de -100°C à 300°C et mieux de -50° à 100°C, de préférence de -10°C à 50°C.

Dans le cadre de la présente invention, on ajoute aux particules de polymère en dispersion une quantité suffisante d'un composé particulier, de manière à abaisser la Tg du film de polymère et améliorer l'adhérence du film de polymère sur son support, en particulier les matières kératiniques. Le composé plastifiant abaisse notamment la température de transition vitreuse du polymère d'au moins 2, 3 ou 4 °C, de préférence de 5°C à 20°C. Dans un mode de réalisation préféré, le composé plastifiant abaisse notamment la température de transition vitreuse du polymère d'au moins 2, 3 ou 4 °C, de préférence de 5°C à 20°C, lorsque le composé plastifiant représente au plus 10% en poids du polymère.

Selon un mode de mise en oeuvre, le composé a un paramètre de solubilité $\delta_h$ compris entre 5,5 et 11, de préférence entre 5,9 et 11, de préférence entre 7 et 10,5, de préférence entre 9 et 10, de préférence encore entre 8 et 10 $(J/cm^3)^{1/2}$.

Le paramètre de solubilité $\delta_P$ du composé est de préférence compris entre 1,5 et 4,5, de préférence entre 1,5 et 4, de préférence entre 1,5 et 3,5, de préférence encore entre 2 et 3 $(J/cm^3)^{1/2}$.

Le composé est de préférence liquide à température ambiante et pression atmosphérique. Il est par exemple le phosphate de tributoxyéthyle.

La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190.

Selon cet espace de HANSEN :

- $\delta_P$ caractérise les forces d'interactions de DEBYE entre dipôles permanents ; et
- $\delta_h$ caractérise les forces d'interactions spécifiques (type liaisons hydrogène, acide/base, donneur/accepteur, etc.) ;

Les paramètres $\delta_P$ et $\delta_h$ sont généralement exprimés en $(J/cm^3)^{½}$. Ils sont déterminés à température ambiante (25°C) et en particulier selon la méthode de calcul indiquée dans le document brevet de KAO ci-dessus.

Dans la composition selon l'invention, on peut utiliser un mélange de composés satisfaisant aux relations ci-dessus. Dans ce cas, les paramètres de solubilité du mélange sont déterminés à partir de ceux des composés pris séparément, selon les relations suivantes:

$$\delta_{pmel} = \sum_i xi\, \delta_{pi} \qquad \text{et} \qquad \delta_{hmel} = \sum_i xi\, \delta_{hi}$$

où xi représente la fraction volumique du composé (i) dans le mélange.

Il est à la portée de l'homme du métier de déterminer les quantités de chaque composé pour obtenir un mélange de composés répondant aux relations ci-dessus.

Dans ce mode de réalisation, le composé peu être choisi parmi les esters d'au moins un acide carboxylique comprenant 1 à 7 atomes de carbones et d'un polyol comprenant au moins 4 groupes hydroxyles.

Le polyol selon l'invention peut être un ose - polyhydroxyaldéhyde (aldose) ou polyhydroxycétone (cétose) - cyclisé ou non. Le polyol est de préférence un ose cyclisé sous forme d'hémi-acétal.

Le polyol peut également être un polyol dérivé d'un ose, comme l'érythritol, le xylitol ou le sorbitol.

Parmi les aldoses, on peut citer le D-ribose, le D-xylose, le L-arabinose, le D-glucose (ou alpha-D-glucopyranose lorsqu'il est sous forme d'hémi-acétal cyclique), le D-mannose, et le D-galactose.

Parmi les cétoses, on peut citer le D-xylulose et le D-fructose (ou béta-D-fructofuranose lorsqu'il est sous forme d'hémi-acétal cyclique).

Le polyol peut être un mono- ou un polysaccharide comprenant un à 10 oses, de préférence de un à 4, de préférence encore un ou deux oses. Le polyol peut être choisi parmi l'érythritol, le xylitol, le sorbitol, le glucose, et le saccharose.

Le polyol selon l'invention est de préférence un disaccharide. Parmi les disaccharides, on peut citer le saccharose (alpha-D-glucopyranosyl-(1-2)-béta-D-fructofuranose), le lactose (béta-D-galactopyranosyl-(1-4)-béta-D-glucopyranose) et le maltose (alpha-D-glucopyranosyl-(1-4)-béta-D-glucopyranose).

Le polyol peut être un polysaccharide constitué de plusieurs oses identiques ou d'au moins deux oses différents. L'ester selon l'invention peut être constitué d'un polyol substitué par au moins deux acides monocarboxyliques différents, ou par au moins trois acides monocarboxyliques différents.

L'ester peut être obtenu par copolymérisation de deux esters selon l'invention, en particulier par copolymérisation i) d'un saccharose substitué par des groupements benzoyle et ii) d'un saccharose substitué par des groupements acétyle et/ou isobutyryle.

L'acide est de préférence un acide monocarboxylique choisi en particulier parmi les acides comprenant 1 à 7 atomes de carbones, de préférence 1 à 5 atomes de carbone, par exemple les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique et l'acide benzoïque.

Selon un mode de mise en oeuvre préféré, l'ester est le di-acétate-hexa-(2-méthyl-propanoate) de saccharose.

Selon un autre mode de mise en oeuvre, le composé selon l'invention est choisi parmi les esters des acides polycarboxyliques aliphatiques ou aromatiques et des alcools aliphatiques ou aromatiques comprenant de 1 à 10 atomes de carbone.

Les alcools aliphatiques ou aromatiques comprennent de 1 à 10 atomes de carbone, de préférence de 1 à 8, par exemple de 1 à 6. Il peuvent être choisis parmi les alcools R1OH, tels que R1 représente méthyle, éthyle, propyle, isopropyle, butyle, hexyle, éthylhexyle, décyle, isodécyle, ou benzyle substitué par un alkyle comprenant 1 à 6 atomes de carbone.

Les acides polycarboxyliques aliphatiques ou aromatiques comprennent de préférence 3 à 12 atomes de carbone, de préférence de 3 à 10 atomes de carbone, de préférence de 3 à 8 atomes de carbone par exemple 6 ou 8 atomes de carbones.

Les acides polycarboxyliques aliphatiques ou aromatiques sont avantageusement des acides dicarboxyliques ou des acides tricarboxyliques.

Parmi les acides dicarboxyliques, on peut citer ceux qui correspondent à la formule $HOOC-(CH_2)_n-COOH$, telle que n est compris entre 1 et 10, de préférence entre 2 et 8, par exemple 2, 4, 6 ou 8.

On préfère les acides dicarboxyliques choisis parmi les acides succiniques, adipiques et sébaciques.

Les acides dicarboxyliques peuvent aussi être choisis parmi l'acide phthalique et ses dérivés, en particulier le phthalate de butylbenzyle, le phthalate de dibutyle, le phthalate de diéthylhexyle, le phthalate de diéthyle et le phthalate de diméthyle.

Parmi les acides tricarboxyliques, on peut citer les triacides qui correspondent à la formule

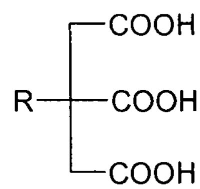

dans laquelle R représente un radical hydrocarboné, de préférence H, -OH ou -OCOR' tel que R' représente un alkyle comprenant de préférence de 1 à 6 atomes de carbone.

Les acides tricarboxyliques sont notamment choisis parmi l'acide acétyl-citrique et ses dérivés.

Parmi les citrates, on peut citer l'acétyl-citrate de tributyle, l'acétyl-citrate de triéthyle, l'acétyl-citrate de triéthylhexyle, l'acétyl-citrate de trihexyle, le butyroyl- citrate trihexyle, le citrate d'isodécyle, le citrate d'isopropyle, le citrate de tributyle et le citrate de triéthylhexyle. Selon un mode de mise en oeuvre, l'acide carboxylique n'est pas le citrate d'acétyl tributyle.

Parmi les adipates, on peut citer l'adipate de dibutyle et l'adipate de diéthyl-2-hexyle. Selon un mode de mise en oeuvre, le composé selon l'invention n'est pas l'adipate de diisopropyle.

Parmi les sébaçates, on peut citer le sébaçate de dibutyle, le sébaçate de diéthylhexyle, le sébaçate de diéthyle et le sébaçate de diisopropyle.

Parmi les succinates, on peut citer le succinate de diéthylhexyle et le succinate de diéthyle.

Le composé est de préférence choisi parmi les composés ayant une masse moléculaire inférieure à 5000, de préférence inférieure à 2000, de préférence inférieure à 1000, dé préférence encore inférieure à 900 g/mol. La masse moléculaire du composé est avantageusement supérieure à 100 g/mol.

Le composé ne comprend de préférence aucun groupe polaire, en particulier aucun groupe hydroxyle. Les "groupes polaires" sont par exemple des groupes polaires ioniques
ou non ioniques choisis parmi -COOH ; -OH ; oxyde d'éthylène; oxyde de propylène;-$PO_4$ ; -NHR ; -$NR_1R_2$ avec $R_1$, $R_2$ formant éventuellement un cycle et représentant un radical alkyle ou alkoxy linéaire ou ramifié en $C_1$ à $C_{20}$.

Le composé est avantageusement présent en une quantité comprise entre 0,1 et 25% en poids, de préférence entre 0,5 et 15% en poids, de préférence encore entre 3 et 15% en poids.

Le rapport massique entre les particules de polymère et le composé est avantageusement compris entre 0,5 et 100, de préférence entre 1 et 50, de préférence entre 1 et 10, de préférence encore entre 1 à 5.

**Stabilisant des particules de polymère**

Les particules de polymère sont de préférence stabilisées en surface grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquences, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquences, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les

copolymères greffés à squelette poly(méthylacrylate de méthyle) et à greffons polyisobutylène.

Comme copolymères blocs greffés ou séquences comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylèné/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse du polymère est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciaie. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquences comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

**Phase grasse**

Généralités

La phase grasse liquide de la composition peut être constituée de toute huile cosmétiquement ou dermatologiquement acceptable, et de façon générale physiologiquement acceptable, notamment choisie parmi les huiles d'origine minérale, végétale ou synthétique, carbonées, hydrocarbonées, fluorées et/ou siliconées, seules ou en mélange dans la mesure où elles forment un mélange homogène et stable macroscopiquement et où elles sont compatibles avec l'utilisation envisagée.

La phase grasse liquide totale de la composition peut représenter de 5 % à 90 % du poids total de la composition et de préférence de 20 à 85 %. Avantageusement, elle représente au moins 30 % du poids total de la composition. De préférence, cette phase grasse contient au moins une huile volatile.

Huiles volatiles de la phase grasse

Selon l'invention, on utilise avantageusement une ou plusieurs huiles volatiles.

Ces huiles peuvent être des huiles hydrocarbonées ou des huiles siliconées comportant éventuellement des groupements alkyle ou alkoxy pendants ou en bout de chaîne siliconée.

Comme huile de silicone volatile utilisable dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme autre huile volatile utilisable dans l'invention, on préfère notamment les huiles isoalcanes (appelées

aussi isoparaffines) en $C_8$-$C_{16}$ comme l'isododécane, l'isodécane, l'isohexadécane et par exemple les huiles vendus sous les noms commerciaux d'ISO-PAR, de PERMETHYL et notamment l'isododécane (PERMETHYL 99 A).

Les huiles volatiles représentent notamment de 5 à 85 % du poids total de la composition, et mieux de 20 à 75 %.

Solvant de synthèse des particules de polymère

La dispersion de polymère peut être fabriquée comme décrit dans le document EP-A-749747.

On prépare un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit un solvant de synthèse tel que les monomères initiaux, et l'amorceur radicalaire, y sont solubles, et les particules de polymère obtenues y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

La phase volatile de la composition peut être constituée par ou comprendre le solvant de synthèse des particules de polymères dispersées.

Huile non volatile de la phase grasse

La phase grasse contient de préférence au moins une huile non volatile apolaire ou peu polaire.

Comme huile non volatile peu polaire utilisable dans l'invention, on peut citer les apolaires ou, notamment les huiles comportant une chaîne alkyle notamment en $C_3$-$C_{40}$. Comme exemple d'huile apolaires ou peu polaires, on peut citer :

- les hydrocarbures linéaires ou ramifiés tels que les huiles de paraffine, de vaseline et de naphthalène légères, le polyisobutène hydrogéné ;
- les huiles hydrocarbonées d'origine animale comme le squalène ;
- les huiles hydrocarbonées végétales telles que les triglycérides liquides d'acides gras d'au moins 10 atomes de carbone;
- les esters et les éthers de synthèse notamment d'acides gras comme les huiles de formule $R_1(CO)_xOR_2$ dans laquelle $R_1$ représente le reste d'un acide comportant de 2 à 29 atomes de carbone avec x valant 0 ou 1 et $R_2$ représente une chaîne hydrocarbonée contenant de 3 à 30 atomes de carbone comme par exemple l'acétyl-citrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tri-décyle, le dodécane di-oléate ou le stéarate de di-iso-cétyle, le propionate d'arachidyle, le dibutyl phthalate, le carbonate de propylène, le pentanoate d'octyldodécyle ; les esters de polyol comme la vitamine F, l'isostéarate de sorbitane, le triisostéarate de glycérine ou de diglycérine ;
- les huiles siliconées telles que les polydiméthylsiloxanes (PDMS), comportant éventuellement une chaîne alkyle ou alcoxy en $C_3$-$C_{40}$ ou une chaîne phénylée telle que les phényltriméthicones, les polyalkylméthylsiloxanes, éventuellement fluorés comme les polyméthyltrifluoro-propyldiméthylsiloxanes, ou par des groupements fonctionnels tels que des groupements hydroxyle, thiol et/ou aminé ; les polysiloxanes modifiés par des acides gras, des alcools gras ou des polyoxyalkylènes,
- les huiles fluorées;
- leurs mélanges.

Ces huiles apolaires ou peu polaires non volatiles peuvent représenter de 0,1 à 20 % du poids total de la composition, mieux de 0,5 à 10 %.et mieux encore de 1 à 5% du poids total de la composition.

De préférence, l'huile non volatile est apolaire. Elle est avantageusement choisie parmi les hydrocarbures notamment les alcanes comme le polyisobutène hydrogéné.

La phase grasse peut également contenir une huile polaire choisie parmi les esters d'acide gras de 7 à 29 atomes de carbone comme le malate de diisostéaryle, le palmitate d'isopropyle, l'adipate de diisopropyle, les triglycérides des acides caprylique/caprique comme ceux vendus par la société Stearineries Dubois ou ceux vendus sous les dénominations Miglyol 810, 812 et 818 par la société Dynamit Nobel, l'huile de beurre de karité, le myristate d'isopropyle, le stéarate de butyle, le laurate d'hexyle, l'adipate de diisopropyle, l'isononate d'isononyle, le laurate de 2-hexyldécyle, le palmitate de 2-octyl-décyle, le myristate ou le lactate de 2-octyl-dodécyle, le succinate de 2-diéthyl-hexyle, le palmitate d'éthyl-2hexyle, le stéarate d'octyl-2-dodécyle, l'huile de ricin ; les esters d'acide lanolique, d'acide laurique, d'acide stéarique ; les alcools gras supérieurs (de 7 à 29 atomes de carbone) tels que l'alcool stéarylique, l'alcool linoléique ou linolénique, l'alcool isostéarique, l'octyl 2-dodécanol, le décanol, le dodécanol, l'octadécanol ou l'alcool oléique ; les acides gras supérieurs (de 7 à 29 atomes de carbone) tels que l'acide myristique, l'acide palmitique, l'acide stéarique, l'acide béhénique, l'acide oléique, l'acide linoléique, l'acide linolénique ou l'acide isostéarique ; leurs mélanges.

Ces huiles polaires non volatiles peuvent représenter de 0,1 à 10 % du poids total de la composition et mieux de 1 à 5 %.

Selon un mode particulier de l'invention, la phase grasse liquide contient au moins une huile volatile apolaire et au moins une huile non volatile apolaire.

## Agent gélifiant

Selon un mode de réalisation de l'invention, la première composition peut comprendre un agent gélifiant de la phase grasse liquide, lequel est un gélifiant polymère ou minéral.

L'agent gélifiant augmente la viscosité de la phase grasse liquide ou la rigidifie. L'agent gélifiant selon la présente invention ne couvre pas les cires.

L'agent gélifiant peut aussi être choisi parmi les homopolymères ou copolymères de l'éthylène dont la masse moléculaire en poids est comprise entre 300 et 500 000, et mieux entre 500 et 100 000.
L'agent gélifiant peut être choisi parmi les copolymères d'oléfines à cristallisation controlée tels que décrits dans la demande EP-A-1 034 776 du demandeur comme par exemple le copolymère d'éthylène/octène vendu sous la référence Engage 8400 par la Société Dupont de Nemours.
Ce ou ces copolymères d'oléfine sont utilisés par exemple à des concentrations de 0,5 à 20 % et mieux de 1 à 10% du poids total de la première composition.

Selon un autre mode de mise en oeuvre, on utilise une polycaprolactone comme agent gélifiant.

En particulier la polycaprolactone est choisie parmi les homopolymères d'ε-caprolactones. L'homopolymérisation peut être initiée avec un diol, notamment un diol ayant de 2 à 10 atomes, tels que le diéthylène glycol, le 1,4-butanediol, le néopentyl glycol.

On peut utiliser par exemple les polycaprolactones de poids moléculaire compris 300 entre 2000, notamment celles commercialisées sous le nom de CAPA ® 2125 par la société SOLVAY.

La polycaprolactone peut être présente dans la composition en une teneur allant de 0,1 % à 30 % en poids, par rapport au poids total de la composition, de préférence de 0,5 % à 25 % en poids, et mieux de 1 % à 20 % en poids, voire de 3 % à 15 % en poids.

Selon un autre mode de mise en oeuvre, le gélifiant présent dans la composition selon l'invention est un polymère amorphe formé par polymérisation d'une oléfine tel que décrit dans la demande EP 1 002 528 dont le contenu est incorporé par référence dans la présente demande. L'oléfine peut être notamment un monomère à insaturation éthylénique élastomérique.
Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

Par polymère amorphe, on entend un polymère qui n'a pas de forme cristalline.

Le gélifiant polymérique amorphe peut être notamment un copolymère dibloc, tribloc, multibloc, radial, étoile, ou leurs mélanges, de préférence un copolymère tribloc, multibloc, radial, étoile, et leurs mélanges.
De tels gélifiants polymérique sont décrits dans la demande US-A-2002/005562 et dans le brevet US-A-5 221 534
Avantageusement, le gélifiant polymère est un copolymère bloc amorphe de styrène et d'oléfine.
Le gélifiant polymère est de préférence hydrogéné pour réduire les insaturations éthyléniques résiduelles après la polymérisation des monomères.

En particulier, le gélifiant polymère est un copolymère, éventuellement hydrogéné, à blocs styrène et à blocs éthylène/alkylène en $C_3$-$C_4$.
Le gélifiant polymérique est avantageusement un copolymère tribloc, de préférence hydrogéné, choisi parmi les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650E, Kraton® G1652, Kraton® D1101, Kraton® D1102, Kraton® D1160 par la société Kraton.

Le gélifiant polymérique amorphe peut être présent en une teneur allant de 0,05 % à 5 % en poids, par rapport au poids total de la composition, et de préférence allant de 0,1 % à 3 % en poids, et préférentiellement allant de 0,2 % à 2 % en poids.

Avantageusement, les particules de polymère dispersées dans la phase organique et le gélifiant polymère sont présents dans la composition en une teneur telle que le rapport pondéral / particules de polymère dispersées et stabilisées en surface / gélifiant polymère va de 10 à 30, de préférence de 15 à 25, et de préférence encore de 18 à 22.

Selon un autre mode de mise en oeuvre, l'agent gélifiant peut être notamment choisi parmi :

- les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium ;
- la silice pyrogénée éventuellement traitée hydrophobe en surface dont la taille des particules est inférieure à 10 micron ; et leurs mélanges.

Comme gélifiant de phase organique, on peut encore citer :

- les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ;
- les galactommananes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-Hance-AG ;
- les gommes notamment siliconées comme les PDMS ayant une viscosité supérieure 100 000 cSt et leurs mélanges ;

## Dispersion colloïdale

La composition selon l'invention contient de préférence des particules solides à température ambiante, dispersées dans le milieu physiologiquement acceptable introduites dans la composition sous forme d'une dispersion colloïdale, appelé aussi "pâte particulaire", telle que décrite dans la demande WO 02/39961 dont le contenu est incorporé par référence dans la présente demande.

Par dispersion colloïdale ou "pâte particulaire", on entend au sens de l'invention une dispersion colloïdale concentrée de particules enrobées ou non dans un milieu continu, stabilisée à l'aide d'un agent dispersant ou éventuellement sans agent dispersant. Ces particules peuvent être choisies parmi les pigments, les nacres, les charges solides et leurs mélanges. Ces particules peuvent être de toute forme notamment de forme sphérique ou allongée comme des fibres. Elles sont insolubles dans le milieu.

L'agent dispersant sert à protéger les particules dispersées contre leur agglomération ou floculation. La concentration en dispersant généralement utilisée pour stabiliser une dispersion colloïdale est de 0,3 à 5 $mg/m^2$, de préférence de 0,5 à 4 $mg/m^2$, de surface de particules. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

La dispersion colloïdale est une suspension de particules de taille généralement micronique (<10 μm) dans un milieu continu. La fraction volumique de particules dans une dispersion concentrée est de 20 % à 40 %, de préférence supérieure à 30 %, ce qui correspond à une teneur pondérale pouvant aller jusqu'à 70 % selon la densité des particules.

Les particules dispersées dans le milieu peuvent être constituées de particules minérales ou organiques ou de leurs mélanges tels que ceux décrits ci-après.

Le milieu continu de la pâte peut être quelconque et contenir tout solvant ou corps gras liquide et leurs mélanges. Avantageusement, le milieu liquide de la pâte particulaire est l'un des corps gras liquides ou huiles que l'on souhaite utiliser dans la composition, faisant ainsi partie de la phase grasse liquide.

De façon avantageuse, la "pâte particulaire" ou dispersion colloïdale est une "pâte pigmentaire" contenant une dispersion colloïdale de particules colorées, enrobées ou non. Ces particules colorées sont des pigments, des nacres ou un mélange de pigments et/ou de nacres.

Avantageusement, la dispersion colloïdale représente de 0,5 à 60 % en poids de la composition et mieux de 2 à 40 % et encore mieux de 2 à 30 %.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune ou rouge ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille. Les pigments peuvent représenter de 0,1 à 50 % en matière active et notamment de 0,5 à 35 % et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 25 % (en matière active) du poids total de la composition et mieux de 0,1 à 15 % (si présents). On peut ainsi utiliser des pigments à propriétés goniochromatiques et des pigments à reflets métalliques tels que décrits dans la demande déposée sous le numéro FR 0 209 246, dont le contenu est incorporé dans la présente demande par référence.

Les charges peuvent être minérales ou organiques, lamellaires ou sphériques. On peut citer le talc, le mica, la silice, le kaolin, les poudres de Nylon® (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses telles que l'Expancel® (Nobel Industrie), le Polytrap® (Dow Corning) et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads@ de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

## Cire

La composition selon l'invention peut, de plus, comprendre, selon le type d'application envisagée, au moins une cire.

Une cire, au sens de la présente invention, est un composé gras lipophile, solide à température ambiante (25°C), à changement d'état solide/liquide réversible, ayant une température de fusion supérieure à 40°C pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. C'est cette recristallisation dans le mélange qui est responsable de la diminution de la brillance dudit mélange.

On préfère dans le cadre de la présente invention des cires hydrocarbonées linéaires. Leur point de fusion est avantageusement supérieur à 35°C, par exemple supérieur à 55°C, de préférence supérieur à 80°C.

Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.

Les cires hydrocarbonées linéaires incluent les polymères et copolymères de l'éthylène, de poids moléculaire

compris entre 400 et 800, par exemple la Polywax 500 ou Polywax 400 commercialisée par New Phase Technologies.

Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies

Les cires peuvent être présentes à raison de 2-30% en poids dans la composition et mieux de 5 à 20%, de préférence entre 5 et 15%, en vue de ne pas trop diminuer la brillance de la composition et du film déposé sur les lèvres et/ou la peau.

**Additifs et galéniques**

La composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la composition.

La composition peut comprendre, en outre, tout autre additif usuellement utilisé dans de telles compositions, tel que de l'eau, des antioxydants, des parfums, des conservateurs et des huiles essentielles.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide, ou de liquide appliqués au moyen d'un tube ou du doigt. Elles peuvent alors constituées des fonds de teint ou des rouges à lèvres, des produits solaires ou de coloration de la peau.

La composition de l'invention est avantageusement anhydre et contiennent dans ce cas moins de 5 % d'eau par rapport au poids total de la composition.

Ces compositions à application topique peuvent constituer notamment une composition cosmétique, dermatologique, hygiénique ou pharmaceutique de protection, de traitement
ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

La composition selon l'invention peut se présenter sous la forme d'une composition dermatologique ou de soin de la peau et/ou des phanères ou sous forme d'une composition de protection solaire, d'hygiène corporelle, notamment sous forme de déodorant. Elle se présente alors notamment sous forme non colorée. Elle peut alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

Les compositions selon l'invention sont de préférence des rouges à lèvres sous la forme d'un stick ou sous forme fluide.

Bien entendu la composition de l'invention doit être cosmétiquement ou dermatologiquement acceptable, à savoir contenir un milieu physiologiquement acceptable non toxique et susceptible d'être appliqué sur la peau, les phanères ou les lèvres du visage d'êtres humains. Par cosmétiquement acceptable, on entend au sens de l'invention une composition d'aspect, d'odeur et de toucher agréables.

**Produit de maquillage bi-couche**

La présente invention a également pour objet un produit cosmétique de maquillage contenant une première et une seconde compositions, la première composition étant constituée par la composition décrite précédemment, et la seconde composition comprenant un second milieu physiologiquement acceptable.

Par produit de maquillage, on entend un produit contenant un agent colorant permettant le dépôt d'une couleur sur une matière kératinique (la peau, les lèvres ou les phanères) de personne humaine par l'application sur la matière kératinique de produits tels que des rouges à lèvres, des fards, des eye-liners, des fonds de teint ou des autobronzants,

des produits de maquillage semi permanent (tatouage).

Lorsque le produit de maquillage est destiné au maquillage des lèvres, la première composition peut être sous la forme d'un stick ou sous forme fluide et la deuxième composition peut également être sous la forme d'un stick ou sous forme fluide. Un produit de maquillage des lèvres selon l'invention comprend de préférence une première composition sous la forme d'un stick, la deuxième composition pouvant être sous forme d'un stick ou sous forme fluide conditionné dans un tube. Le produit selon l'invention comprend deux (ou plusieurs) compositions physiologiquement acceptables conditionnées séparément ou ensemble dans un même article de conditionnement ou dans deux (ou plusieurs) articles de conditionnement séparés ou distincts.

Selon un mode de mise en oeuvre , ces compositions sont conditionnées séparément et avantageusement, dans un même article de conditionnement comprenant deux embouts.

L'objet de la présente invention est donc en particulier un produit cosmétique de maquillage se présentant sous la forme d'un fond de teint, d'un fard à joues ou à paupières, d'un rouge à lèvres, d'un produit ayant notamment des propriétés de soin, d'un eye-liner, d'un produit anti-cernes, ou d'un produit de maquillage du corps (du type tatouage). Le produit cosmétique de maquillage est en particulier un rouge à lèvres.

L'invention a aussi pour objet un kit de maquillage contenant un produit cosmétique de maquillage tel que défini précédemment, dans lequel les différentes compositions sont conditionnées séparément et sont accompagnées de moyens d'application appropriés. Ces moyens peuvent être des. pinceaux, des brosses, des stylos, des crayons, des feutres, des plumes, des éponges, des tubes et /ou des mousses. On peut également appliquer la composition au doigt.

La première composition du produit selon l'invention peut être appliquée sur la matière kératinique pour constituer une couche de base, sur laquelle on dépose la seconde composition pour obtenir une couche de dessus ou surcouche ou couche de finition. Il est possible d'appliquer sous la couche de base une sous couche ayant la constitution ou non de la seconde couche.

Il est aussi possible de déposer une deuxième surcouche sur la première surcouche ayant une constitution identique ou non à celle de la première surcouche. De préférence, le maquillage obtenu est un maquillage comprenant une couche de base et une surcouche.

En particulier première composition est un fond de teint, un fard, un rouge à lèvres, un brillant à lèvres, un eye liner, un produit de maquillage du corps, et la deuxième composition est un produit de soin ou un produit destiné à préserver ou améliorer les propriétés cosmétiques de la première composition, en particulier la brillance et le confort.

L'invention se rapporte aussi à un procédé de maquillage de la peau et/ou des lèvres et/ou des phanères consistant à appliquer sur la peau et/ou les lèvres et/ou les phanères un produit cosmétique de maquillage tel que défini précédemment.

L'invention a encore pour objet un procédé de maquillage de la peau et/ou des lèvres et/ou des phanères d'être humain, consistant à appliquer sur la peau, les lèvres et/ou les phanères une première couche d'une première composition comprenant, dans un milieu physiologiquement acceptable, des particules de polymère dispersées dans une phase organique liquide et un agent de coloration, puis à appliquer, sur tout ou partie de la première couche, une seconde couche d'une seconde composition comprenant un milieu physiologiquement acceptable.

Plus précisément, le procédé selon l'invention consiste à appliquer sur la peau, les lèvres et/ou les phanères d'être humain une première couche d'une première composition comprenant, dans un premier milieu physiologiquement acceptable, des particules de polymère dispersées dans une phase organique liquide et un agent de coloration, à laisser sécher ladite première couche, puis à appliquer, sur tout ou partie de la première couche, une seconde couche d'une seconde composition comprenant un second milieu physiologiquement acceptable.

Ce maquillage bicouche peut être adapté à tous les produits de maquillage de la peau aussi bien du visage que du corps, des muqueuses comme les lèvres, de l'intérieur des paupières inférieures, et des phanères comme les ongles, les cils, les cheveux, les sourcils, voire les poils. La seconde couche peut former des motifs et peut être appliquée avec un stylo, crayon ou tout autre instrument (éponge, doigt, pinceau, brosse, plume etc.). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a aussi pour objet un support maquillé comprenant une première couche d'une première composition comprenant, dans un premier milieu physiologiquement acceptable, des particules de polymère dispersées dans une phase liquide et une seconde couche d'une seconde composition déposée sur tout ou partie de la première couche, comprenant un second milieu physiologiquement acceptable.

**Seconde composition**

Selon un des aspects de l'invention, la deuxième composition selon l'invention comprend au moins un composé apolaire ou peu polaire qui peut être choisi parmi les huiles, les gommes et/ou les cires. La deuxième composition comprend de préférence plus de 70%, de préférence plus de 80% en poids, de préférence 100% en poids de composés

apolaires ou peu polaires. Ces composés apolaires ou peu polaires sont de préférence des composés siliconés, des agents de coloration ou des agents gélifiants.

En particulier, la deuxième composition peut être une des compositions décrites dans la demande US 60/375 814 dont le contenu est incorporé dans la présente demande par référence.

Selon un mode préféré de réalisation de l'invention, le milieu physiologiquement acceptable de la seconde composition comprend une phase liquide non volatile à température ambiante et pression atmosphérique.

Phase liquide non volatile de la deuxième composition

La deuxième composition comprend avantageusement de 15 à 90% en poids d'au moins une phase liquide non volatile.

Par « phase liquide non volatile », on entend tout milieu susceptible de rester sur la peau ou les lèvres pendant plusieurs heures. Une phase liquide non volatile a en particulier une pression de vapeur à température ambiante et pression atmosphérique, non nulle inférieure à 0,02 mm de Hg (2,66 Pa) et mieux inférieure à $10^{-3}$ mm de Hg (0,13 Pa).

La phase liquide non volatile de la seconde composition est de préférence apolaire ou peu polaire et comprend de préférence au moins une huile hydrocarbonée, une huile siliconée et/ou une huile fluorée liquide.

Les huiles non volatiles peuvent être choisies parmi les polydiméthylsiloxanes; les alkyldimé thicones; les polyphénylméthylsiloxanes tels que les phényldiméthicones et les phényltrimé thicones; ainsi que les silicones modifiés par des groupements aliphatiques et/ou aromati ques, éventuellement fluorés, ou par des groupements fonctionnels tels que des groupe ments hydroxyles, thiols et/ou amines.

On préfère comme huile non volatile, une huile choisie parmi les silicones de formule (I):

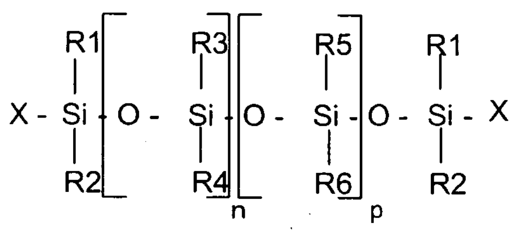

(I)

dans laquelle :

- $R_1$, $R_2$, $R_5$ et $R_6$ sont, ensemble ou séparément, un radical alkyle ayant 1 à 6 atomes de carbone,
- $R_3$ et $R_4$ sont, ensemble ou séparément, un radical alkyle ayant de 1 à 6 atomes de carbone, ou un radical aryle,
- X est un radical alkyle ayant de 1 à 6 atomes de carbone, un radical hydroxyle ou un radical vinyle,
- n et p étant choisis de manière à conférer à l'huile une masse moléculaire en poids inférieure à 200 000 g/mol , de préférence inférieure à 150 000 g/mol et de préférence encore inférieure à 100 000 g/mol.

On choisit par exemple comme l'huile silicone non volatile de formule (1) une polydiméthylsiloxane de viscosité comprise entre entre 0,5 et 60 000 cSt, de préférence entre 0,5 et 10 000 cSt, de préférence entre 0,5 et 1 000 cSt, de préférence encore la DC 200 de viscosité 350 cSt, commercialisée par Dow Corning.

Selon un mode de mise en oeuvre, l'huile silicone non volatile de formule (1) est une polydiméthylsiloxane de viscosité comprise de préférence entre 0,5 et 500 cSt, de préférence encore entre 1 et 10 cSt.

De préférence, la phase liquide non volatile de la seconde composition comprend au moins une huile fluorée de formule (II) :

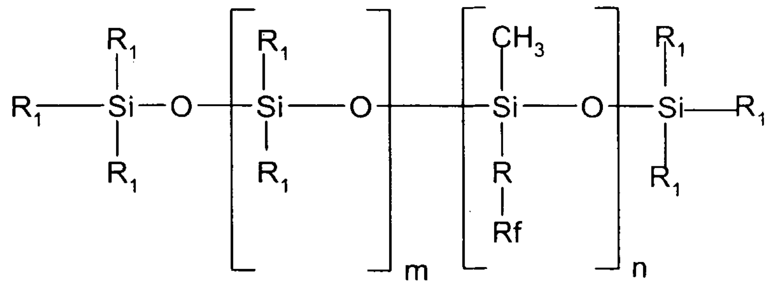

(II)

dans laquelle :

- R représente un groupement alkylényle linéaire ou ramifié, ayant 1 à 6 atomes de carbone, de préférence un groupement divalent méthylényle, éthylényle, propylényle ou butylényle,
- Rf représente un radical fluoroalkyle, notamment un radical perfluoroalkyle, ayant 1 à 9 atomes de carbone, de préférence 1 à 4 atomes de carbone,
- $R_1$ représentent, indépendamment l'un de l'autre, un radical alkyle en $C_1$-$C_{20}$, de préférence en $C_1$-$C_4$, un radical hydroxyle, ou un radical phényle,
- m est choisi de 0 à 150, de préférence de 20 à 100, et
- n est choisi de 1 à 300, de préférence de 1 à 100.

On préfère les huiles de formule (II) telles que R1 est un méthyle, R est un éthyle et Rf est CF3. On peut notamment citer comme composés fluorosiliconés de formule (II) ceux qui sont commercialisés par la société Shin Etsu sous les dénominations 'X22-819', 'X22-820', 'X22-821' et 'X22-822' ou encore 'FL-1 00'.

On peut également citer parmi les huiles fluorées, les polyéthers fluorés choisis parmi les composé de formule (III) suivante :

$$R_6\text{- }(CF_2\text{-}CFR_3\text{-}CF_20)_p\text{ - }(CFR_4\text{-}CF_2\text{-}O)q\text{ - }(CFR_5\text{-}O)_r\text{ - }R_7 \qquad \text{(III)}$$

dans laquelle :

- $R_3$ à $R_6$ représentent, de manière indépendante l'un de l'autre, un radical monovalent choisi parmi -F, -$(CF_2)$n-$CF_3$, et -O-$(CF_2)$n-$CF_3$,
- $R_7$ représente un radical monovalent choisi parmi -F et -$(CF_2)$n-$CF_3$,
- avec n allant de 0 à 4 inclus,
- p allant de 0 à 600, q allant de 0 à 860, r allant de 0 à 1500, et p, q et r étant des entiers choisis de manière à ce que la masse moléculaire en poids du composé va de 500 à 100000, de préférence de 500 à 10000.

Les huiles fluorées peuvent également être choisies parmi les alcanes fluorés sont choisi parmi les perfluoroalcanes et les fluoroalcanes en C2-C50, de préférence en C5-C30, tels que la perfluorodécaline, le perfluoroadamantane et le bromoperfluorooctyle et leurs mélanges.

La deuxième composition peut contenir un polymère siliconé de haut poids moléculaire.

Lorsque la deuxième composition selon l'invention est liquide, elle comprend avantageusement 20 à 50 % en poids d'un polymère siliconé de haut poids moléculaire.

Lorsque la deuxième composition selon l'invention est solide, elle comprend avantageusement 2 à 40 % en poids d'un polymère siliconé de haut poids moléculaire.

Ce polymère peut être liquide ou solide à température ambiante et sa masse moléculaire en poids est supérieure ou égale à 200 000 g/mol, de préférence compris entre 200 000 et 2 500 000, de préférence entre 200 000 et 2 000 000 g/mol.

La viscosité de ce polymère siliconé peut être comprise entre 100 000 et 5 000 000 cSt, de préférence entre 100 000 et 1 000 000 cSt, de préférence encore entre 300 000 et 700 000 cSt, mesurée selon la norme ASTM D-445.

Le polymère siliconé de haut poids moléculaire est avantageusement un polymère non greffé, c'est à dire un polymère obtenu par polymérisation d'au moins un monomère, sans réaction subséquente des chaînes latérales avec un autre composé chimique. Le polymère siliconé est de préférence choisi parmi les diméthiconols, les fluorosilicones,

les diméthicones et leurs mélanges. Le polymère est de préférence un homopolymère.

En particulier, on peut utiliser un polymère siliconé de haut poids moléculaire répondant à la formule (IV):

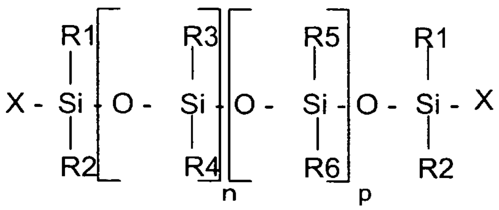

(IV)

dans laquelle :
dans laquelle :

$R_1$, $R_2$, $R_5$ et $R_6$ sont, ensemble ou séparément, un radical alkyle ayant 1 à 6 atomes de carbone, éventuellement substitué par au moins un atome de fluor,
$R_3$ et $R_4$ sont, ensemble ou séparément, un radical alkyle ayant de 1 à 6 atomes de carbone ou un radical aryle,
X est un radical alkyle ayant de 1 à 6 atomes de carbone, un radical hydroxyle, un radical vinyle, allyle ou un radical alkoxyle ayant de 1 à 6 atomes de carbone,
n et p étant choisis de manière à ce que le composé siliconé ait une masse moléculaire en poids supérieure ou égale à 200 000 g/mol.
p est de préférence égal à 0.

Les polymères de formule (IV) tels $R_1$ à $R_6$ représentent un groupement méthyle et le substituant X représente un groupement hydroxyle, sont des diméthiconols. On cite par exemple les polymères de formule (III) tels que p=0 et n est compris entre 2 000 et 40 000, de préférence entre 3 000 et 30 000.

Les polymères ont notamment une masse moléculaire comprise entre 1 500 000 et 2 000 000 g/mol.

Selon un mode de mise en oeuvre, le polymère siliconé de haut poids moléculaire est la diméthiconol commercialisée par Dow Corning dans une polydiméthylsiloxane (5 cSt) sous la référence D2-9085, la viscosité du mélange étant égale à 1 550 cSt, ou la diméthiconol commercialisée par Dow Corning dans l'octaméthylcyclotétrasiloxane sous la référence DC 1503. On préfère également la diméthiconol (de poids moléculaire égal à 1 770 000 g/mol) commercialisée par Dow Corning sous la référence Q2-1403 ou Q2-1401, la viscosité du mélange étant égale à 4 000 cSt.

Comme polymère siliconé de haut poids moléculaire de formule (IV)utilisable selon l'invention, on peut citer ceux pour lesquels :

- les substituants $R_1$ à $R_6$ et X représentent un groupement méthyle, comme celle vendue sous la dénomination SE30 par la société Général Electric, et celle vendue sous la dénomination AK 500000 par la société Waker,
- les substituants $R_1$ à $R_6$ et X représentent un groupement méthyle, p et n sont tels que le poids moléculaire est de 250 000 g/mol, comme celle vendue sous la dénomination Silbione 70047 V par la société Rhodia,
- les substituants $R_1$ à $R_6$ représentent un groupement méthyle, le substituant X représente un groupement hydroxyle, comme celle vendue sous la dénomination Q2-1401 ou Q2-1403 par la société Dow Corning,
- les substituants $R_1$, $R_2$, $R_5$, $R_6$ et X représentent un groupement méthyle, les substituants $R_3$ et $R_4$ représentent un groupement aryle, n et p sont tels que le poids moléculaire du polymère soit de 600 000 g/mol, comme celle vendue sous la dénomination 761 par la société Rhône-Poulenc.

Le polymère siliconé de haut poids moléculaire est de préférence introduit dans la composition sous la forme d'un mélange avec une silicone liquide, la viscosité de ladite silicone liquide étant comprise entre 0,5 et 10 000 cSt, de préférence entre 0,5 et 500 cSt, de préférence encore entre 1 et 10 cSt.
La silicone fluide peut être choisie parmi les polyalkylsiloxanes, les polyarylsiloxanes, les polyalkylarylsiloxanes et leurs mélanges. La silicone liquide peut être une silicone volatile telle qu'une polydiméthylsiloxane cyclique comprenant 3 à 7 motifs $-(CH_3)_2SiO-$.
La silicone liquide peut également être une silicone non volatile polydiméthylsiloxane, notamment de viscosité comprise entre 0,5 et 10 000 cSt, de préférence encore de l'ordre de 5 cSt, par exemple la silicone commercialisée sous la référence DC 200 par Dow Corning.
La proportion du polymère siliconé de haut poids moléculaire dans le mélange polymère siliconé de haut poids moléculaire/silicone liquide est de préférence compris entre 10/90 et 20/80. La viscosité du mélange polymère siliconé de haut poids moléculaire/silicone liquide est de préférence comprise entre 1 000 et 10 000 cSt.

Les diméthicones de haut poids moléculaire selon l'invention incluent les diméthicones décrites dans le brevet US 4 152 416. Elles sont par exemple commercialisées sous les références SE30, SE33, SE 54 et SE 76.

Les diméthicones selon l'invention sont par exemple des composés de formule (IV) telle que $R_1$ à $R_6$ et X sont des méthyles et p=0. Le poids moléculaire de ces polymères est de préférence compris entre 200 000 et 300 000, de préférence entre 240 000 et 260 000 g/mol.

Les diméthicones selon l'invention incluent les polydiméthylsiloxanes, les copolymères (polydiméthylsiloxane)(méthylvinylsiloxane), les copolymères poly(diméthylsiloxane)(diphényl)(méthylvinylsiloxane), et leurs mélanges

Les fluorosilicones de haut poids moléculaire selon l'invention ont de préférence un poids moléculaire compris entre 200 000 et 300 000, de préférence entre 240 000 et 260 000 g/mol.

Cire dans la deuxième composition

La deuxième composition contient avantageusement au moins une cire, notamment lorsqu'elle est sous forme solide.

Les cires peuvent être présentes à raison de 2-30% en poids dans la composition et mieux de 5 à 20%, de préférence entre 5 et 15%, de la composition.

On préfère dans le cadre de la présente invention des cires hydrocarbonées linéaires. Leur point de fusion est avantageusement supérieur à 35°C, par exemple supérieur à 55°C, de préférence supérieur à 80°C.
Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.
Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.
Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies.

La deuxième composition contient avantageusement une cire siliconée, telle qu'une diméthicone comprenant des groupements alkyles en bout de chaîne. Ces groupements alkyles ont de préférence plus de 18 atomes de carbones, de préférence entre 20 et 50, de préférence entre 30 et 45 atomes de carbone.

La cire siliconée répond par exemple à la formule (V) ou (VI)

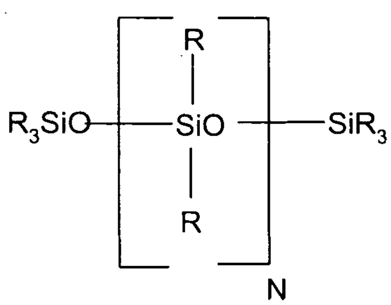

(V)

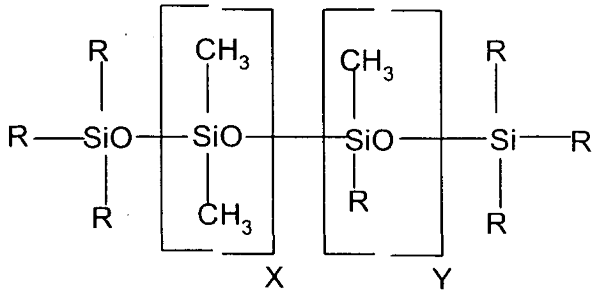

(VI)

dans lesquelles R est un alkyle, X est égal ou supérieur à zéro, et N et X sont égaux ou supérieurs à un. R comprend de 1 à 50 atomes de carbone à la condition que le compose soit solide à température ambiante.

Des exemples de cires siliconées sont par exemple

- Les C20-24 alkyl méthicone, C24-28 alkyl diméthicone, C20-24 alkyl diméthicone, C24-28 alkyl diméthicone commercialisées par Archimica Fine Chemicals sous la référence SilCare 41 M40, SilCare 41 M50, SilCare 41 M70 and SilCare 4.1 M80,
- Les stéaryl diméthicone de référence SilCare 41 M65 commercialisées par Archimica ou de référence DC-2503 commercialisée par Dow-Corning
- Les stéaroxytriméthylsilane vendues sous la référence SilCare 1 M71 ou DC-580
- Les produits ABIL Wax 9810, 9800, or 2440 de Wacker-Chemie G,mbH,
- Les C30-45 alkyl méthicone commercialisées par Dow Corning sous la référence AMS-C30 Wax, de même que les C30-45 alkyl diméthicone commercialisées sous la référence SF1642 ou SF-1632 par General Electric.

Agent gélifiant

La deuxième composition peut contenir un agent gélifiant tel que décrit précédemment.

Agent de coloration

La deuxième du produit cosmétique de maquillage selon l'invention peut contenir un agent de coloration qui peut être choisi parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la première composition.
Par « nacres », il faut comprendre des particules irisées, notamment produites par certains mollusques dans leur coquille ou bien synthétisées, insolubles dans le milieu de la première composition.
Par « colorants », il faut comprendre des composés généralement organiques solubles dans les corps gras comme les huiles ou dans une phase hydroalcoolique.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.
Les colorants hydrosolubles sont par exemple le jus de betterave, le bleu de méthylène, le caramel.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune, brun ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium
ou aluminium dont celles soumises à une certification par la Food and Drug Administration (FDA) (exemple D&C ou FD&C) et ceux exempts de la certification FDA comme les laques à base de carmin de cochenille.

Les nacres ou pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. On peut ainsi utiliser des pigments à propriétés goniochromatiques, notamment à cristaux liquides ou multicouches, et/ou des pigments à reflets métalliques tels que décrits dans la demande FR 0 209 246 dont le contenu est incorporé dans la présente demande par

référence.

La seconde composition du produit selon l'invention peut également mais pas obligatoirement contenir un agent de coloration.

De manière générale, les agents de coloration représentent de 0,001 à 60 %, et mieux de 0,01 à 50 %, et mieux encore, de 0,1 à 40 % du poids total de chaque première et seconde composition.

L'agent de coloration ou la charge peuvent en outre être présents sous forme de « pâte particulaire ».

Galéniques du produit de maquillage

Chaque composition du produit de maquillage bicouche selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Avantageusement, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition. En particulier, le produit de maquillage bicouche entier est sous forme anhydre.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

De préférence, chaque composition se présente sous la forme d'un stick plus ou moins rigide.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

De préférence, la composition qui est appliquée en première couche est sous forme solide, ce qui permet une application plus pratique, une meilleure stabilité dans le temps et en température de la composition et permet un tracé précis du maquillage, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut être se présenter sous forme, de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Le produit est en particulier un rouge à lèvres.

De préférence, la première et/ou la seconde composition est sous forme solide.

Avantageusement, la couche du dessus présente des propriétés de soin, de brillance et de transparence.

L'invention a encore pour objet un produit à lèvres, un fond de teint, un tatouage, un fard à joues ou à paupières contenant une première et une seconde compositions telles que décrites précédemment.

Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

L'invention est illustrée plus en détail dans les exemples suivants. Les pourcentages sont des pourcentages en poids.

**Exemple 1 : dispersion de polymère**

On prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. On obtient ainsi une dispersion de particules de poly(acrylate de méthyle/acide acrylique) stabilisées en surface dans l'isododécane par un copolymère dibloc séquencé polystyrène/copoly(éhylène-propylène) vendu sous le nom de Kraton G1701, ayant un taux de matière sèche de 25% en poids.

**Exemple 2 : rouge à lèvres**

| | |
|---|---|
| Cire de polyéthylène (Masse moléculaire en poids 500) | 10,5 % |
| Alcools gras linéaires (PERFORMACOL 550 ALCOHOL commercialisé par New Phases Technologies) | 2,5 % |
| Dispersion de l'exemple 1 | 68 % |
| Acetate isobutyrate de saccharose (EASTMAN SAIS commercialisé par EASTMAN CHEMICAL) | 5 % |
| Pâte pigmentaire | 13,5 % |
| Parfum | 0,5 % |

Dans un poêlon on introduit la cire de polyéthylène, les alcools en C30-50 et la pâte pigmentaire que l'on chauffe à 100°C sous agitation magnétique afin d'obtenir un mélange homogène. La pâte pigmentaire est constituée de 70 % de pigments, 1 % de stéarate d'acide poly(12-hydroxystéarique) et 29 % de polyisobutène hydrogéné.
On ajoute ensuite la dispersion selon l'exemple 1 et l'acétate isobutyrate de saccharose tout en maintenant la température et l'agitation. La composition est coulée dans des moules à 40°C qui sont placés à -20°C pendant trente minutes. On procède ensuite au démoulage des sticks. Les sticks obtenus sont homogènes en teinte et glissant à l'application. Ils conduisent à un dépôt sur les lèvres de bonne tenue, ne migrant pas, non transfert, ne collant pas.

**Exemple 3 : rouge à lèvres**

| | |
|---|---|
| Polystyrène/poly(éthykène-propylène) KRATON G-1650E (commercialisé par la société Kraton) | 0,5 % |
| Dispersion de l'exemple 1 | 65 % |
| Polycaprolactone (CAPA 2125 fabriqué par SOLVAY) | 10 % |
| Acétate isobutyrate de saccharose (EASTMAN SAIB commercialisé par EASTMAN CHEMICAL) | 10 % |
| Pâte pigmentaire | 13,5 % |
| Parfum | qs |
| Conservateur | qs |

On fait fondre la polycaprolactone à 100°C puis on lui ajoute sous agitation la pâte pigmentaire, dont la composition est identique à celle de l'exemple 2. On ajoute ensuite les autres ingrédients jusqu'à obtention d'un mélange homogène. Le mélange est ensuite re- . froidi à température ambiante et conditionner dans des bouillottes.

**Exemple 4 : deuxième composition liquide**

| | |
|---|---|
| POLY DIMETHYLSILOXANE commercialisée sous la référence Silbione 70047 V par Rhodia (500 000 cSt - 250 000 g/mol) | 40 % |
| POLY DIMETHYLSILOXANE commercialisée par la société Dow Corning sous la référence DC200 (5 cSt) | 60 % |

On mélange les deux ingrédients à 70°C l'aide d'un agitateur de type Raynerie.

**Exemple 5 : deuxième composition solide**

| | % en poids |
|---|---|
| Huile de silicone (PDMS) DC200 de Dow Corning (5 cSt) | 25 |
| DIMETHICONE (and) DIMETHICONOL | 61 |
| D2-9085 de Dow Corning (1550 cSt) TRIFLUOROPROPYL DIMETHICONE (100 cSt) X22-819 de Shin Etsu | 1 |

(suite)

| | % en poids |
|---|---|
| C30-45 ALKYL DIMETHICONE (SF 1642 de GE Bayer Silicone) | 5 |
| Cire de POLYETHYLENE (PM en poids 500) | 8 |

L'huile de silicone, la diméthiconol et la diméthicone fluorée sont mélangées à chaud jusqu'à former un mélange homogène. On ajoute ensuite la $C_{30}$-$C_{45}$ alkyl diméthicone dans le mélange précédent porté à 110°C. La cire de polyéthylène est ensuite ajoutée peu à peu jusqu'à obtention d'un mélange homogène. Le mélange est refroidi à 90-95°C puis versé dans les moules, qui sont placés à -20°C pendant trente minutes. On procède enfin au démoulage des sticks.

## Revendications

1. Composition cosmétique comprenant a) au moins une phase grasse liquide et b) une dispersion de particules de polymère dispersées dans ladite phase grasse, **caractérisée en ce qu'**elle contient en outre c) au moins un composé choisi parmi les esters des acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère.

2. Composition selon la revendication 1, **caractérisée en ce que** les acides polycarboxyliques aliphatiques ou aromatiques comprennent de 3 à 12 atomes de carbone, de préférence de 3 à 10 atomes de carbone, de préférence de 3 à 8 atomes de carbone par exemple 6 ou 8 atomes de carbones.

3. Composition selon la revendication 1 ou 2, **caractérisée en ce que** les alcools aliphatiques ou aromatiques comprennent de 1 à 10 atomes de carbone, de préférence de 1 à 8, par exemple de 1 à 6, et peuvent être choisis parmi les alcools R1OH, tels que R1 représente méthyle, éthyle, propyle, isopropyle, butyle, hexyle, éthylhexyle, décyle, isodécyle, ou benzyle substitué par un alkyle comprenant 1 à 6 atomes de carbone.

4. Composition selon l'une des revendications précédentes, **caractérisée en ce que** les acides sont choisis parmi les acides dicarboxyliques correspondant à la formule HOOC-(CH2)n-COOH, telle que n est compris entre 1 et 10, de préférence entre 2 et 8, par exemple 2, 4, 6 ou 8.

5. Composition selon la revendication précédente, **caractérisée en ce que** les acides dicarboxyliques sont choisis parmi les acides succiniques, adipiques et sébaciques.

6. Composition selon la revendication précédente, **caractérisée en ce que** le composé est choisi parmi le succinate de diéthylhexyle et le succinate de diéthyle.

7. Composition selon la revendication 5, **caractérisée en ce que** le composé n'est pas l'adipate de diisopropyle.

8. Composition selon la revendication 5, **caractérisée en ce que** le composé est choisi parmi l'adipate de dibutyle et l'adipate de diéthyl-2-hexyle.

9. Composition selon la revendication 5, **caractérisée en ce que** le composé est choisi parmi le sébaçate de dibutyle, le sébaçate de diéthylhexyle, le sébaçate de diéthyle et le sébaçate de diisopropyle.

10. Composition selon la revendication 1, **caractérisée en ce que** les acides polycarboxyliques sont choisis parmi l'acide phthalique et ses dérivés, en particulier le phthalate de butylbenzyle, le phthalate de dibutyle, le phthalate de diéthylhexyle, le phthalate de diéthyle et le phthalate de diméthyle.

11. Composition selon l'une des revendications précédentes, **caractérisée en ce que** les acides polycarboxylilques sont choisis parmi les acides tricarboxyliques correspondent à la formule

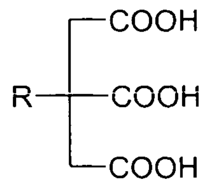

dans laquelle R représente un radical hydrocarboné, de préférence H, -OH où -OCOR' tel que R' représente un alkyle comprenant de préférence de 1 à 6 atomes de carbone.

**12.** Composition selon la revendication 11, **caractérisée en ce que** les acides tricarboxyliques sont choisis parmi l'acide acétyl-citrique et ses dérivés.

**13.** Composition selon la revendication 12, **caractérisée en ce que** le composé n'est pas l'acétyl-citrate de tributyle.

**14.** Composition selon la revendication 12, **caractérisée en ce que** le composé est choisi parmi l'acétyl-citrate de triéthyle, l'acétyl-citrate de triéthylhexyle, l'acétyl-citrate de trihexyle, le butyroyl- citrate trihexyle, le citrate d'isodécyle, le citrate d'isopropyle, le citrate de tributyle et le citrate de triéthylhexyle.

**15.** Composition selon l'une des revendications précédentes, **caractérisée en ce que** le composé ne comprend aucun groupe polaire choisi parmi les groupes polaires ioniques ou non ioniques tels que -COOH ; -OH ; oxyde d'éthylène; oxyde de propylène; $-PO_4$;-NHR ; $-NR_1R_2$ avec $R_1$, $R_2$ formant éventuellement un cycle et représentant un radical alkyle ou alkoxy linéaire ou ramifié en $C_1$ à $C_{20}$.

**16.** Composition selon l'une des revendications précédentes, **caractérisée en ce que** le composé a un paramètre de solubilité $\delta_h$ compris entre 5,5 et 11, de préférence entre 5,9 et 11, de préférence entre 7 et 10,5, de préférence entre 8 et 10 de préférence encore entre 9 et 10 $(J/cm^3)^{1/2}$.

**17.** Composition selon l'une des revendications précédentes, **caractérisée en ce que** le composé a un paramètre de solubilité $\delta_P$ compris entre 1,5 et 4,5, de préférence entre 1,5 et 4, de préférence entre 1,5 et 3,5, de préférence encore entre 2 et 3 $(J/cm^3)^{1/2}$.

**18.** Composition selon l'une des revendications précédentes, **caractérisée en ce que** le composé est présent en une quantité comprise entre 0,1 et 25% en poids, de préférence entre 0,5 et 15% en poids, de préférence encore entre 3 et 10% en poids.

**19.** Composition selon l'une des revendications précédentes, **caractérisée en ce que** le rapport massique entre les particules de polymère et le composé est compris entre 0,5 et 100, de préférence entre 1 et 50, de préférence entre 1 et 10, de préférence encore entre 1 et 5.

**20.** Composition selon l'une des revendications précédentes, **caractérisée en ce que** les particules de polymère sont insolubles dans les alcools hydrosolubles.

**21.** Composition selon l'une des revendications précédentes, **caractérisée en ce que** les particules de polymère sont choisies parmi les polyuréthanes, polyuréthanes-acryliques, polyurées, polyurée/polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

**22.** Composition selon l'une des revendications précédentes, **caractérisée en ce que** le polymère est filmifiable.

**23.** Composition selon l'une des revendications précédentes, **caractérisée en ce que** le polymère représente, en matière sèche, de 2 à 40% du poids total de la première composition, de préférence de 5 à 30% et mieux encore de 8 à 20%.

**24.** Composition selon l'une des revendications précédentes, **caractérisée en ce que** les particules de polymère sont

stabilisées en surface dans ladite phase grasse liquide par un stabilisant choisi parmi les polymères séquences, les polymères greffés, les polymères statistiques, et leurs mélanges.

25. Composition selon la revendication 22 **caractérisée en ce que** le stabilisant est un polymère bloc greffé ou séquencé, comprenant au moins un bloc résultant de la polymérisation de diène et au moins un bloc d'un polymère vinylique.

26. Composition selon la revendication précédente, **caractérisée en ce que** le stabilisant est un polymère dibloc.

27. Composition selon l'une des revendications précédentes, **caractérisée en ce qu'**elle comprend une dispersion colloïdale de particules solides à température ambiante choisies parmi les pigments, des nacres et leurs mélanges.

28. Composition selon la revendication précédente, **caractérisée en ce que** la dispersion colloïdale représente de 0,5 à 60 % en poids de la composition et mieux de 2 à 40 % et mieux encore de 2 à 50 %.

29. Composition selon l'une des revendications 27 ou 28, **caractérisée en ce que** la dispersion colloïdale comprend un dispersant des particules choisi parmi le stéarate de l'acide poly(12-hydroxystéarique), l'acide poly (12-hydroxystéarique), le 2-dipolyhydroxystéarate de diglycéryle et leurs mélanges.

30. Composition selon l'une des revendications 27 à 29, **caractérisée en ce que** la dispersion colloïdale contient un corps gras liquide à température ambiante.

31. Composition selon la revendication 30, **caractérisée en ce que** le corps gras liquide à température ambiante est le polyisobutène hydrogéné.

32. Composition selon l'une des revendications précédentes, dans laquelle la phase grasse liquide comprend une phase grasse non volatile et une phase grasse volatile.

33. Composition selon la revendication précédente, **caractérisée en ce que** la phase grasse liquide volatile contient au moins une huile choisie parmi les isoalcanes en $C_8$-$C_{16}$.

34. Composition selon la revendication précédente, **caractérisée en ce que** la phase grasse liquide volatile contient de l'isododécane ou l'isohexadécane.

35. Composition selon l'une des revendications précédentes, **caractérisée en ce qu'**elle est exempte d'huile siliconée.

36. Composition selon l'une des revendications précédentes, **caractérisée en ce qu'**elle est exempte d'alcool gras.

37. Composition selon la revendication 32, dans laquelle la phase grasse liquide non volatile est apolaire.

38. Composition selon la revendication 33, **caractérisée en ce que** la phase grasse liquide non volatile contient une huile hydrocarbonée, par exemple le polyisobutène hydrogéné.

39. Composition selon l'une quelconque des revendications précédentes, **caractérisée par le fait qu'**elle contient un agent gélifiant choisi parmi les gélifiants polymères et les gélifiants minéraux.

40. Composition selon la revendication précédente, **caractérisée par le fait** l'agent gélifiant est un agent gélifiant polymère choisi parmi les copolymères blocs amorphes de styrène et d'oléfine.

41. Composition selon la revendication précédente, **caractérisée par le fait que** le gélifiant polymèrique est avantageusement un copolymère tribloc, de préférence hydrogéné, choisi parmi les copolymères de styrène-éthylène/propylène-styrène, les copolymères de styrène-éthylène/butadiène-styrène, les copolymères de styrène-isoprène-styrène, les copolymères de styrène-butadiène-styrène.

42. Composition selon l'une quelconque des revendications 39 à 41, **caractérisée par le fait que** le gélifiant est présent en une teneur allant de 0,1 % à 5 % en poids, par rapport au poids total de la composition, et de préférence allant de 0,2 % à 3 % en poids, et préférentiellement allant de 0,5 % à 2 % en poids.

**43.** Composition selon la revendication 39, **caractérisée par le fait** l'agent gélifiant est un agent gélifiant polymère choisi parmi les polycaprolactones.

**44.** Composition selon la revendication précédente, **caractérisée par le fait** les polycaprolactones sont choisies parmi les homopolymères d'ε-caprolactones, de poids moléculaire compris 300 entre 2000 g/mol.

**45.** Composition selon l'une des revendications précédentes, **caractérisée en ce qu'**elle contient au moins une cire choisie parmi les polymères et copolymères de l'éthylène, les alcools linéaires comprenant 20 à 50 atomes de carbones et leurs mélanges.

**46.** Composition selon l'une des revendications précédentes, se présentant sous forme d'un stick ou bâton, sous forme d'une pâte souple, ou sous forme d'un liquide.

**47.** Composition selon l'une des revendications précédentes, se présentant sous forme anhydre.

**48.** Composition selon l'une des revendications précédentes, se présentant sous la forme d'un produit de soin et/ou de maquillage de la peau et/ou des lèvres.

**49.** Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

**50.** Composition cosmétique comprenant a) au moins une phase grasse liquide, b) une dispersion de particules de polymère dispersées dans ladite phase grasse, **caractérisée en ce qu'**elle content en outre c) au moins un composé ayant un paramètre de solubilité $\delta_h$ compris entre 5,5 et 11 $(J/cm^3)^{½}$, ledit composé étant présent en une quantité suffisante pour plastifier ledit polymère.

**51.** Composition selon la revendication précédente, **caractérisée en ce que** le paramètre de solubilité $\delta_h$ du composé est compris entre 5,9 et 11, de préférence entre 7 et 10,5, de préférence entre 8 et 10 de préférence encore entre 9 et 10 $(J/cm^3)^{1/2}$.

**52.** Composition selon la revendication 50 ou 51, **caractérisée en ce que** le composé a une masse moléculaire inférieure à 5000, de préférence 2000, de préférence inférieure à 1000, de préférence encore inférieure à 900 g/mol.

**53.** Composition selon l'une des revendications 50 à 52, **caractérisée en ce que** le composé ne comprend aucun groupe polaire choisi parmi les groupes polaires ioniques ou non ioniques tels que -COOH ; -OH ; oxyde d'éthylène; oxyde de propylène; $-PO_4$ ; -NHR ; $-NR_1R_2$ avec $R_1$, $R_2$ formant éventuellement un cycle et représentant un radical alkyle ou alkoxy linéaire ou ramifié en $C_1$ à $C_{20}$.

**54.** Composition selon l'une des revendications 50 à 53, **caractérisée en ce que** le paramètre de solubilité $\delta_P$ du composé est compris entre 1,5 et 4,5, de préférence entre 1,5 et 4, de préférence entre 1,5 et 3,5, de préférence encore entre 2 et 3 $(J/cm^3)^{1/2}$.

**55.** Composition selon l'une des revendications 50 à 54, **caractérisée en ce que** le composé est présent en une quantité comprise entre 0,1 et 25% en poids, de préférence entre 0,5 et 15% en poids, de préférence encore entre 3 et 10% en poids.

**56.** Composition selon l'une des revendications 50 à 55, **caractérisée en ce que** le rapport massique entre les particules de polymère et le composé est compris entre 0,5 et 100, de préférence entre 1 et 50, de préférence entre 1 et 10, de préférence encore entre 1 et 5.

**57.** Composition selon l'une des revendications 50 à 56, **caractérisée en ce que** le composé est choisi parmi l'acétyl-citrate de triéthyle, l'acétyl-citrate de triéthylhexyle, l'acétyl-citrate de trihexyle, le butyroyl- citrate trihexyle, le citrate d'isodécyle, le citrate d'isopropyle, le citrate de tributyle et le citrate de triéthylhexyle.

**58.** Composition l'une des revendications 50 à 56, **caractérisée en ce que** le composé n'est pas l'acétyl-citrate de tributyle.

**59.** Composition selon l'une des revendications 50 à 56, **caractérisée en ce que** le composé est choisi parmi le phthalate de butylbenzyle, le phthalate de dibutyle, le phthalate de diéthylhexyle, le phthalate de diéthyle et de le phthalate diméthyle.

**60.** Composition selon l'une des revendications 50 à 56, **caractérisée en ce que** le composé est choisi parmi l'adipate de dibutyle et l'adipate de diéthyl-2-hexyle.

**61.** Composition selon l'une des revendications 50 à 56, **caractérisée en ce que** le composé n'est pas l'adipate de diisopropyle.

**62.** Composition selon l'une des revendications 50 à 56, **caractérisée en ce que** le composé est choisi parmi le sébaçate de dibutyle, le sébaçate de diéthylhexyle, le sébaçate de diéthyle et le sébaçate de diisopropyle.

**63.** Composition selon l'une des revendications 50 à 56, **caractérisée en ce que** le composé est choisi parmi le succinate de diéthylhexyle et le succinate de diéthyle

**64.** Procédé de soin cosmétique ou de maquillage des lèvres ou de la peau, consistant à appliquer sur respectivement les lèvres ou la peau une composition cosmétique telle que définie aux revendications précédentes.

**65.** Procédé pour limiter le transfert et/ou augmenter la tenue et/ou faciliter le dépôt d'une composition de maquillage ou de soin de la peau ou des lèvres, ladite composition contenant une phase grasse liquide et des particules de polymère dispersées dans la phase grasse liquide, ledit procédé consistant à introduire dans la phase grasse liquide au moins un composé ayant un paramètre de solubilité $\delta_h$ compris entre 5,5 et 11 $(J/cm^3)^{1/2}$, ledit composé étant présent en une quantité suffisante pour plastifier ledit polymère.

**66.** Procédé pour limiter le transfert et/ou augmenter la tenue et/ou faciliter le dépôt d'une composition de maquillage ou de soin de la peau ou des lèvres, ladite composition contenant une phase grasse liquide et des particules de polymère dispersées dans la phase grasse liquide, ledit procédé consistant à introduire dans la phase grasse liquide au moins un composé choisi parmi les esters des acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère.

**67.** Utilisation dans une composition à application sur la peau, les lèvres et sur les phanères, de particules d'au moins un polymère dispersées dans une phase grasse liquide et stabilisées en surface par un agent stabilisant et d'au moins un composé ayant un paramètre de solubilité $\delta_h$ compris entre 5,5 et 11 $(J/cm^3)^{1/2}$, ledit composé étant présent en une quantité suffisante pour plastifier ledit polymère., pour limiter le transfert et/ou augmenter sa tenue dans le temps, et/ou faciliter son dépôt.

**68.** Utilisation dans une composition à application sur la peau, les lèvres et sur les phanères, de particules d'au moins un polymère dispersées dans une phase grasse liquide et stabilisées en surface par un agent stabilisant et d'au moins un composé choisi parmi les esters des acides polycarboxyliques aliphatiques ou aromatiques et des alcools aliphatiques ou aromatiques comprenant de 1 à 10 atomes de carbone, ledit composé étant présent en une quantité suffisante pour plastifier ledit polymère, pour limiter le transfert et/ou augmenter sa tenue dans le temps, et/ou faciliter son dépôt.

**69.** Produit cosmétique de maquillage contenant une première et une seconde compositions, la première composition étant constitué d'une composition selon l'une des revendications 1 à 49 ou d'une composition selon l'une des revendications 50 à 63, et la seconde composition comprenant un second milieu physiologiquement acceptable.

Office européen des brevets

# RAPPORT DE RECHERCHE EUROPEENNE

Numéro de la demande
EP 04 29 0462

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| E | WO 2004/028490 A (FERRARI VERONIQUE ; BLIN XAVIER (FR); OREAL (FR)) 8 avril 2004 (2004-04-08)<br>* exemple 2 *<br>----- | 1-69 | A61K7/02<br>A61K7/48<br>A61K7/025 |
| X | WO 02/36083 A (KOLODZIEJ RICHARD ; SIMON JEAN CHRISTOPHE (FR); OREAL (FR); SOISTIER N) 10 mai 2002 (2002-05-10)<br>* page 14, ligne 32 - page 15, ligne 9 *<br>----- | 1-18, 20-22, 32-34, 37,46, 48-55, 57-66 | |
| D,X | EP 1 249 223 A (OREAL) 16 octobre 2002 (2002-10-16)<br>* revendications; exemples *<br>----- | 1-6, 15-34, 37-39, 42, 45-56, 63,64, 67-69 | |
| | | | DOMAINES TECHNIQUES RECHERCHES (Int.Cl.7) |
| X | EP 0 749 747 A (OREAL) 27 décembre 1996 (1996-12-27)<br>* colonne 4, ligne 23-34 *<br>-----<br>-/-- | 1-5, 15-18, 22, 24-26, 32,35, 37, 39-41, 45-54,64 | A61K<br>A61Q |

Le présent rapport a été établi pour toutes les revendications

| Lieu de la recherche | Date d'achèvement de la recherche | Examinateur |
|---|---|---|
| La Haye | 22 septembre 2004 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 04 29 0462

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 1 044 673 A (OREAL) 18 octobre 2000 (2000-10-18)<br>* revendications 1,7,8 *<br>----- | 1-7, 10-17, 20-26, 32-34, 37, 40-42, 45-54, 63,64, 67,68 | |
| X | EP 0 987 012 A (OREAL) 22 mars 2000 (2000-03-22)<br>* revendications 1,7; exemples *<br>----- | 1-6, 15-18, 20-26, 32-34, 37-42, 45-54, 63,64, 67,68 | |
| X | FR 2 798 061 A (OREAL) 9 mars 2001 (2001-03-09)<br>* page 3, ligne 28 - ligne 41; revendications 1,8,15 *<br>----- | 1-6, 15-28, 30-34, 37-39, 45, 50-56, 63-66,68 | DOMAINES TECHNIQUES RECHERCHES (Int.Cl.7) |
| A | FR 2 794 970 A (OREAL) 22 décembre 2000 (2000-12-22)<br>* revendications 1,7,14 *<br>----- | 1-6,16, 18-23, 32,37, 40,41, 45-50,64 | |
| | -/-- | | |

Le présent rapport a été établi pour toutes les revendications

| Lieu de la recherche | Date d'achèvement de la recherche | Examinateur |
|---|---|---|
| La Haye | 22 septembre 2004 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 04 29 0462

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 1 163 897 A (OREAL)<br>19 décembre 2001 (2001-12-19)<br>* revendications; exemples *<br>----- | 1,33,38,<br>43,44 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| La Haye | 22 septembre 2004 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 04 29 0462

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-09-2004

| Document brevet cité au rapport de recherche | Date de publication | Membre(s) de la famille de brevet(s) | Date de publication |
|---|---|---|---|
| WO 2004028490 A | 08-04-2004 | EP 1411069 A2 | 21-04-2004 |
| | | EP 1421928 A2 | 26-05-2004 |
| | | WO 2004028492 A2 | 08-04-2004 |
| | | WO 2004028493 A2 | 08-04-2004 |
| | | WO 2004028488 A2 | 08-04-2004 |
| | | WO 2004028489 A2 | 08-04-2004 |
| | | WO 2004028490 A2 | 08-04-2004 |
| | | WO 2004028491 A2 | 08-04-2004 |
| | | WO 2004028494 A2 | 08-04-2004 |
| | | WO 2004028485 A2 | 08-04-2004 |
| | | WO 2004028486 A2 | 08-04-2004 |
| | | WO 2004028487 A2 | 08-04-2004 |
| | | JP 2004149772 A | 27-05-2004 |
| | | US 2004120906 A1 | 24-06-2004 |
| | | US 2004120920 A1 | 24-06-2004 |
| WO 0236083 A | 10-05-2002 | FR 2815848 A1 | 03-05-2002 |
| | | EP 1333790 A1 | 13-08-2003 |
| | | WO 0236083 A1 | 10-05-2002 |
| | | JP 2004512348 T | 22-04-2004 |
| | | US 2003118531 A1 | 26-06-2003 |
| EP 1249223 A | 16-10-2002 | FR 2823101 A1 | 11-10-2002 |
| | | FR 2823102 A1 | 11-10-2002 |
| | | BR 0201262 A | 11-03-2003 |
| | | CA 2380789 A1 | 10-10-2002 |
| | | CN 1382431 A | 04-12-2002 |
| | | EP 1249223 A1 | 16-10-2002 |
| | | JP 2002322020 A | 08-11-2002 |
| | | US 2003039621 A1 | 27-02-2003 |
| EP 0749747 A | 27-12-1996 | FR 2735689 A1 | 27-12-1996 |
| | | AT 174502 T | 15-01-1999 |
| | | CA 2197496 A1 | 09-01-1997 |
| | | DE 69601147 D1 | 28-01-1999 |
| | | DE 69601147 T2 | 02-06-1999 |
| | | EP 0749747 A1 | 27-12-1996 |
| | | ES 2128149 T3 | 01-05-1999 |
| | | WO 9700663 A1 | 09-01-1997 |
| | | JP 2000044426 A | 15-02-2000 |
| | | JP 3225967 B2 | 05-11-2001 |
| | | JP 10501005 T | 27-01-1998 |
| | | US 5851517 A | 22-12-1998 |
| EP 1044673 A | 18-10-2000 | FR 2792194 A1 | 20-10-2000 |
| | | AT 202919 T | 15-07-2001 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 04 29 0462

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-09-2004

| Document brevet cité au rapport de recherche | Date de publication | Membre(s) de la famille de brevet(s) | Date de publication |
|---|---|---|---|
| EP 1044673 A | | DE 60000004 D1 | 16-08-2001 |
| | | DE 60000004 T2 | 25-04-2002 |
| | | EP 1044673 A1 | 18-10-2000 |
| | | ES 2161676 T3 | 16-12-2001 |
| | | US 6361782 B1 | 26-03-2002 |
| EP 0987012 A | 22-03-2000 | FR 2783415 A1 | 24-03-2000 |
| | | BR 9905295 A | 20-03-2001 |
| | | CA 2281992 A1 | 18-03-2000 |
| | | CN 1249171 A | 05-04-2000 |
| | | EP 0987012 A1 | 22-03-2000 |
| | | JP 2000119124 A | 25-04-2000 |
| | | KR 2000023142 A | 25-04-2000 |
| | | US 6326013 B1 | 04-12-2001 |
| FR 2798061 A | 09-03-2001 | FR 2798061 A1 | 09-03-2001 |
| | | EP 1082953 A1 | 14-03-2001 |
| | | JP 2001089325 A | 03-04-2001 |
| FR 2794970 A | 22-12-2000 | FR 2794970 A1 | 22-12-2000 |
| | | EP 1066819 A1 | 10-01-2001 |
| | | JP 2001039830 A | 13-02-2001 |
| | | US 2004156804 A1 | 12-08-2004 |
| EP 1163897 A | 19-12-2001 | FR 2810239 A1 | 21-12-2001 |
| | | EP 1163897 A1 | 19-12-2001 |
| | | JP 2002020216 A | 23-01-2002 |
| | | US 2002041857 A1 | 11-04-2002 |

EPO FORM P0460

Pour tout renseignement concernant cette annexe : voir Journal Officiel de l'Office européen des brevets, No.12/82